Europäisches Patentamt

European Patent Office

Office européen des brevets

(11) Veröffentlichungsnummer: **0 274 072**
**A1**

---

## (12) EUROPÄISCHE PATENTANMELDUNG

(21) Anmeldenummer: 87118233.3

(22) Anmeldetag: 09.12.87

(51) Int. Cl.⁴: **A61B 6/00**

---

(30) Priorität: 22.12.86 DE 8634355 U

(43) Veröffentlichungstag der Anmeldung:
13.07.88 Patentblatt 88/28

(84) Benannte Vertragsstaaten:
**DE FR GB NL**

(71) Anmelder: **Siemens Aktiengesellschaft Berlin und München**
**Wittelsbacherplatz 2**
**D-8000 München 2(DE)**

(72) Erfinder: **Schäfer, Willi, Dipl.-Ing. (FH)**
**Genglerstrasse 20**
**D-8520 Erlangen(DE)**

---

(54) **Röntgendiagnostikgerät.**

(57) Die Erfindung betrifft ein Röntgendiagnostikgerät mit einem an seinem einen Ende (11) einen Monitor (10) tragenden Tragarm (12) und einem Patientenlagerungstisch (5), wobei der Monitor (10) mittels des Tragarmes (12) relativ zu dem Patientenlagerungstisch (5) verstellbar ist. Der Monitor (10) ist mittels des Tragarmes (12), der mit seinem anderen Ende (13) an einer im Bereich des Fußendes (7) des Patientenlagerungstisches (5) befindlichen Säule (14) gehaltert ist, längs des Patientenlagerungstisches (5) verstellbar und in eine an dessen Fußende (7) befindliche Parkposition verfahrbar.

FIG 1

EP 0 274 072 A1

## Röntgendiagnostikgerät

Die Erfindung betrifft ein Röntgendiagnostikgerät mit einem an seinem einen Ende einen Monitor tragenden Tragarm und einem Patientenlagerungstisch, wobei der Monitor mittels des Tragarmes relativ zu dem Patientenlagerungstisch verstellbar ist.

Ein solches Röntgendiagnostikgerät ist aus der Druckschrift der Firma Siemens "Für schnelles, durchleuchtungsorientiertes Arbeiten im OP, ARCOSKOP 100 OP-II", Bestell-Nr. A19100-M1037-A722-01, bekannt. Bei diesem Röntgendiagnostikgerät ist der Tragarm an der Decke des Untersuchungsraumes bzw. Operationssaales in einer solchen Position befestigt, daß die Bewegungsfreiheit anderer Teile des Röntgendiagnostikgerätes, z.B. eines C-Bogens mit Röntgenröhre und Röntgenbildverstärker, nicht beeinträchtigt ist. Dies hat jedoch zur Folge, daß sich der an dem Tragarm angebrachte Monitor außerhalb der Reichweite einer einen auf dem Patientenlagerungstisch liegenden Patienten unter-suchenden Bedienperson befindet. Außerdem be-findet sich der Monitor nicht im unmittelbaren Blickfeld der Bedienperson, was insbesondere bei Katheterisierungen oder ähnlichen Maßnahmen von Nachteil ist.

Der Erfindung liegt die Aufgabe zugrunde, ein Röntgendiagnostikgerät der eingangs genannten Art so auszubilden, daß sich der Monitor stets in Reichweite und im unmittelbaren Blickfeld einer eine Untersuchung vornehmenden Bedienperson befinden kann, ohne daß damit eine Einschränkung der Einstellbarkeit der übrigen Teile des Röntgendiagnostikgerätes verbunden ist.

Nach der Erfindung wird diese Aufgabe dadurch gelöst, daß der Monitor mittels des Tragarmes, der mit seinem anderen Ende an einer im Bereich des Fußendes des Patientenlagerungstisches be findlichen Säule gehaltert ist, längs des Patientenlagerungstisches verstellbar und in eine an dessen Fußende befindliche Parkposition verfahrbar ist. Der Monitor kann somit unabhängig von der Position, die eine eine Untersuchung vorneh-mende Bedienperson an dem Patientenlagerungsti-sch einnimmt, so positioniert werden, daß er sich in deren Reichweite befindet, wodurch diese den Monitor so einstellen kann, daß er sich jederzeit in ihrem unmittelbaren Blickfeld befindet. Eine Behinderung der Einstellbarkeit der übrigen Teile des Röntgendiagnostikgerätes durch den Monitor bzw. den Tragarm und die diesen tragende Säule ist ausgeschlossen, da sich die Säule im Bereich des Fußendes des Patientenlagerungstisches befindet und der Monitor in eine ebenfalls am Fußende des Patientenlagerungstisches befindliche Parkposition verfahrbar ist. Alle erforderlichen Einstellarbeiten am Röntgendiagnostikgerät können somit ohne Behinderung ausgeführt werden. Außerdem können der Tragarm, die Säule und der in der Parkposition befindliche Monitor beim Betten eines Patienten auf den Patientenlagerungstisch keine Behinderung darstellen. Eine besonders variable Einstellbarkeit des Monitors wird erreicht, wenn der Tragarm um eine im Bereich der Säule befindliche vertikale und/oder horizontale Achse schwenkbar ist.

Eine besonders weitgehende Einstellbarkeit des Monitors wird dann erreicht, wenn der Tragarm einen ersten und einen zweiten Armabschnitt aufweist, die an ihren einen Enden mittels eines Gelenkes um eine vertikale Gelenkachse - schwenkbar miteinander verbunden sind, wobei der erste Armabschnitt mit seinem anderen Ende an der Säule angebracht ist, während der zweite Armabschnitt an seinem anderen Ende den Monitor trägt. Es ist aber auch möglich, daß der erste und der zweite Armabschnitt des Tragarmes an ihren einen Enden mittels eines Gelenkes um eine hori-zontale Gelenkachse schwenkbar miteinander ver- bunden sind, wobei der erste Armabschnitt mit seinem anderen Ende wieder an der Säule angebracht ist, während der zweite Armabschnitt an seinem anderen Ende den Monitor trägt.

Um einen unerwünscht langen Tragarm zu vermeiden, kann vorgesehen sein, daß der Tragarm teleskopartig ausziehbar oder die Säule relativ zu dem Patientenlagerungstisch längsverschiebbar angebracht ist.

Um eine den jeweiligen Bedürfnissen möglichst genau entsprechende Ausrichtung des Monitors zu gestatten, kann vorgesehen sein, daß dieser schwenkbar an dem Tragarm angebracht ist. Aus dem gleichen Grunde ist es von Vorteil, wenn der Tragarm und damit der Monitor relativ zu dem Patientenlagerungstisch höhenverstellbar ist. Außerdem kann vorgesehen sein, daß die Säule, an der der Tragarm gehaltert ist, an dem Patientenlagerungstisch angebracht ist. Falls der Patientenlagerungstisch eine höhenverstellbare Lagerungsplatte besitzt, ist es zweckmäßig, die Säule an der Lagerungsplatte zu befestigen, da der Monitor im Falle einer Höhenverstellung der Lagerungsplatte deren Bewegung folgt.

Um es einer eine Untersuchung durchführenden Bedienperson zu gestatten, von ihrem jeweiligen Standort an dem Patientenlagerungstisch aus bestimmte Bedien-vorgänge an dem Röntgendiagnostikgerät vorzu-nehmen, ist nach einer Ausführung der Erfindung vorgesehen, daß an dem Tragarm ein an seinem einen Ende ein Bedienkästchen tragender Arm angebracht ist, der mit seinem anderen Ende um eine im Bereich des Monitors angeordnete Gelenkverbindung relativ zu dem Tragarm schwenkbar ist. Dabei kann vorgesehen sein, daß der Arm in eine Parkposition schwenkbar ist, in der er sich im wesentlichen parallel zu dem Tragarm erstreckt. Der Arm kann somit, sobald das Bedienkästchen nicht benötigt wird, in die Parkposition gebracht werden, wo er keine Behinderung darstellt.

In den beigefügten Zeichnungen sind Ausführungsbeispiele der Erfindung dargestellt. Es zeigen:

Fig. 1 eine Stirnansicht eines erfindungsgemäßen Röntgendiagnostikgerätes,

Fig. 2 eine Seitenansicht des erfindungsgemäßen Röntgendiagnostikgerätes,

Fig. 3 eine Aufsicht auf das erfindungsgemäße Röntgendiagnostikgerät,

Fig. 4 und 5 unterschiedliche Betriebszustände eines erfindungsgemäßen Röntgendiagnostikgerätes in perspektivischer Darstellung, und

Fig. 6 bis 8 erfindungsgemäße Röntgendiagnostikgeräte in perspektivischer Darstellung.

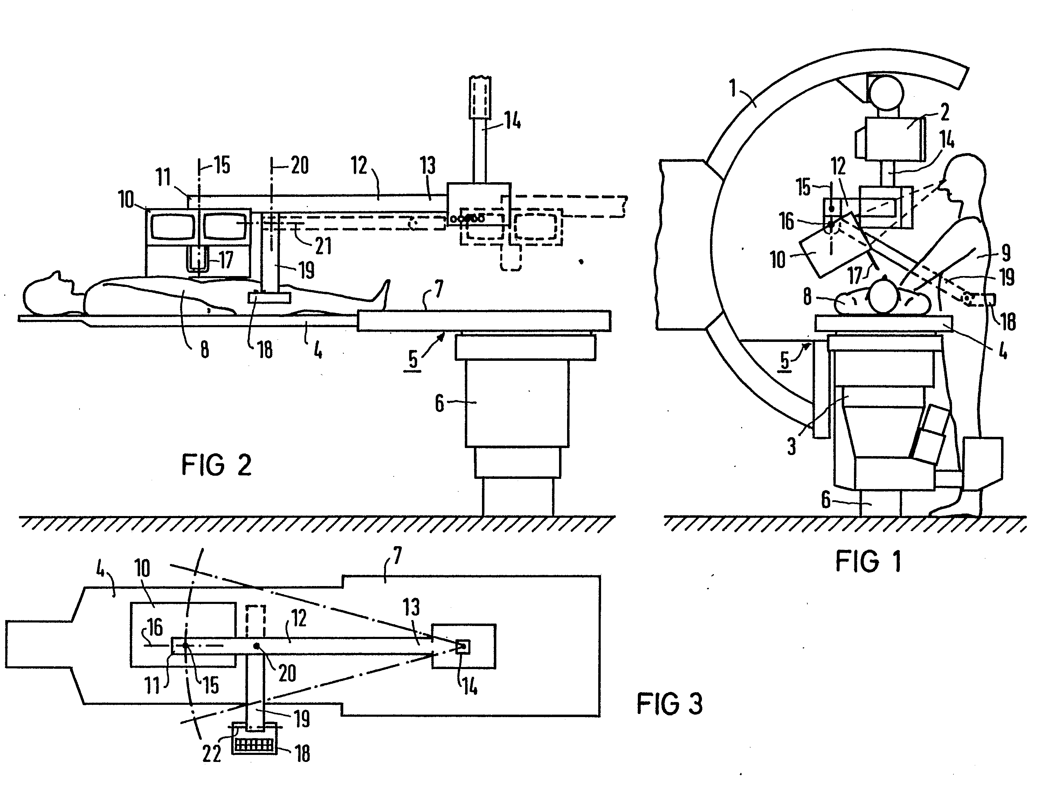

Die Fig. 1 bis 3 zeigen ein erfindungsgemäßes Röntgendiagnostikgerät mit einem frei im Raum einstellbaren C-Bogen 1, der in den Fig. 2 und 3 der Übersichtlichkeit halber nicht dargestellt ist. An diesem sind einander gegenüberliegend ein Röntgenstrahler 2 und ein Röntgenbildverstärker 3 angebracht. Der C-Bogen 1 umgreift die Lagerungsplatte 4 eines Patientenlagerungstisches 5, die an einem teleskopartigen Sockel 6 höhenverstellbar mit ihrem Fußende 7 befestigt ist und einseitig frei auskragt. Der C-Bogen 1 kann somit, wie unter Heranziehung der Fig. 2 ersichtlich ist, längs desjenigen Bereiches der Lagerungsplatte 4, der als Auflage für einen Patienten 8 dient, zur Durchleuchtung beliebiger Bereiche des Körpers des Patienten 8 verschoben werden, ohne mit dem Sockel 6 zu kollidieren. Das Bild des Röntgenbildverstärkers 3 wird mittels einer nicht dargestellten Fernsehkamera und gegebenenfalls einer Bildverarbeitungseinrichtung für eine den Patienten 8 untersuchende Bedienperson 9 sichtbar auf Fernsehmonitoren 10 dargestellt. Diese sind an dem einen Ende 11 eines Tragarmes 12 relativ zu dem Patientenlagerungstisch 5 verstellbar angeordnet.

Der Tragarm 12 ist mit seinem anderen Ende 13 an einer im Bereich des Fußendes 7 des Patientenlagerungstisches 5 vorgesehenen Säule 14 gehalten. Dabei ist diese in nicht näher dargestellter Weise längsverschiebbar an der Decke des Untersuchungsraumes angebracht, und zwar derart, daß die Fernsehmonitore 10 zusammen mit dem sie tragenden Tragarm 12 ausgehend von der in Fig. 2 dargestellten Endposition soweit nach rechts verfahren werden können, daß sie eine in Fig. 2 strichliert eingetragene Parkposition im Bereich des Fußendes 7 des Patientenlagerungstisches 5 einnehmen.

Aus den Fig. 1 bis 3 wird deutlich, daß damit der Bedienperson 9 die Möglichkeit gegeben ist, die Fernsehmonitore 10 stets in ihrer Reichweite und in ihrem unmittelbaren Gesichtsfeld zu positionieren.

Wie den Fig. 1 bis 3 weiter zu entnehmen ist, ist die Säule 14 nach Art eines Teleskops ausgebildet, so daß es möglich ist, die Höhe des Tragarmes 12 und damit der Fernsehmonitore 10 relativ zu der Lagerungsplatte 4 des Patientenlagerungstisches 5 zu verstellen. Da der Tragarm 12 mittels einer nicht näher dargestellten Gelenkverbindung um die vertikal verlaufende Längsachse der Säule 14 schwenkbar an dieser angebracht ist, ist es möglich, die Fernsehmonitore 10 in parallel zur Lagerungsplatte 4 verlaufenden Ebenen zu verstellen (siehe Fig. 3). Die Fernsehmonitore 10 sind mittels einer ebenfalls nicht näher dargestellten Gelenkverbindung um eine vertikale Achse 15 und eine horizontale Achse 16 schwenkbar an dem einen Ende 11 des Tragarmes 12 angebracht. Zusätzlich zu der durch die bereits beschriebene Längsverschiebbarkeit der Säule 14 und die Schwenkbarkeit und Höhenverstellbarkeit des Tragarms 12 gegebenen Einstellmöglichkeiten der Fernsehmonitore 10 wird durch diese Maßnahmen eine den jeweiligen Bedürfnissen der Bedienperson 9 entsprechende Feineinstellbarkeit der Fernsehmonitore 10 gewährleistet, wobei an diesen ein Griffbügel 17 angebracht ist, mit dessen Hilfe die Bedienperson 9 die entsprechenden Einstellbewegungen durchführen kann.

An dem Tragarm 12 ist ein an seinem einen Ende ein Bedienkästchen 18 tragender Arm 19 angebracht, der mit seinem anderen Ende mittels einer im Bereich der Fernsehmonitore 10 befindlichen, nicht näher dargestellten Gelenkverbindung um die vertikale Achse 20 und die horizontale Achse 21 relativ zu dem Tragarm 12 schwenkbar ist. Das Bedienkästchen 18 selbst, das z.B. Bedienelemente zur motorischen Verstellung des Patientenlagerungstisches 5 oder des C-Bogens 1 tragen kann, ist um die horizontale Achse 22 - schwenkbar an dem Arm 19 angebracht, so daß es von der Bedienperson 9 stets in eine dieser genehme Position gebracht werden kann. Falls das Bedienkästchen 18 nicht benötigt wird, kann der Arm 19 in eine Parkposition geschwenkt werden, in der er sich unterhalb des Tragarmes 12 befindet und im wesentlichen parallel zu diesem erstreckt.

Die Fernsehmonitore 10 sowie der Arm 19 sind ausgehend von ihren in den Fig. 1 bis 3 dargestellten Positionen um die vertikale Achse 15 bzw. die vertikale Achse 20 um wenigstens 180° - schwenkbar, so daß die Bedienperson 9 von beiden Seiten des Patientenlagerungstisches 5 aus Handhabungen an dem Patienten 8 über die Fernsehmonitore 10 verfolgen und Bedienvorgänge mittels des Bedienkästchens 18 vornehmen kann.

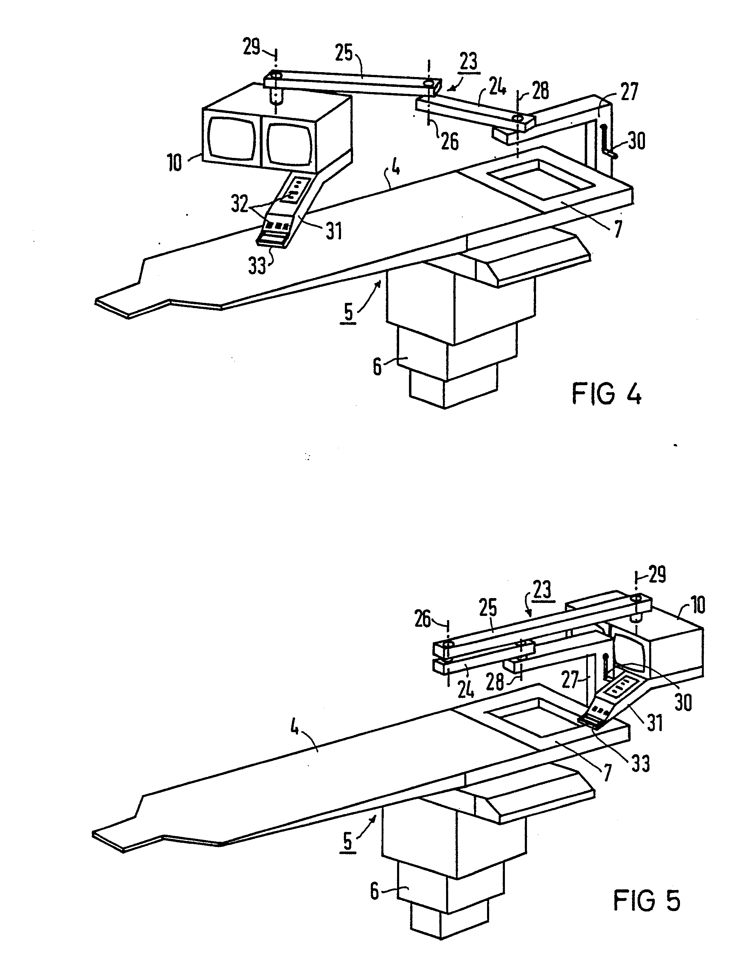

Von dem in den Fig. 4 und 5 dargestellten erfindungsgemäßen Röntgendiagnostikgerät sind der Übersichtlichkeit halber nur der Patientenlagerungstisch 5 und ein die Fernsehmonitore 10 tragender insgesamt mit 23 bezeichneter Tragarm dargestellt. Letzterer weist einen ersten und einen zweiten Armabschnitt 24 bzw. 25 auf, die an ihren einen Enden mittels eines nicht näher dargestellten Gelenkes um eine vertikale Gelenkachse 26 schwenkbar miteinander verbunden sind, wobei der erste Armabschnitt 24 an seinem anderen Ende an einer an der Lagerungsplatte 4 des Patientenlagerungstisches 5 befestigten abgewin kelten Säule 27 um eine vertikale Achse 28 - schwenkbar angebracht ist, während der zweite Armabschnitt 25 an seinem anderen Ende die Fernsehmonitore 10 trägt, die ebenfalls um eine vertikale Achse 29 schwenkbar sind. Dabei sind einerseits die Längen der Armabschnitte 24 und 25 unter Berücksichtigung der Länge des abgewinkelten Teiles der Säule 27 und andererseits die um die vertikale Gelenkachse 26 und die vertikale Achse 28 möglichen Schwenkwinkel so bemessen, daß die Fernsehmonitore 10 in die in Fig. 5 dargestellte Parkposition im Bereich des Fußendes 7 des Patientenlagerungstisches 5 verfahrbar sind. Die Säule 27 ist mittels eines mit einer Handkurbel 30 betägigbaren, nicht näher dargestellten, z.B. als Schraubengetriebe ausgebildeten Mechanismus relativ zu der Lagerungsplatte 4 höhenverstellbar. An den Fernsehmonitoren 10 ist eine Konsole 31 angebracht, die Bedienelemente 32 trägt und an die ein Handgriff 33 angeformt ist, mittels dessen die Fernsehmonitore 10 in eine von einer Bedienperson gewünschte Position gebracht werden können.

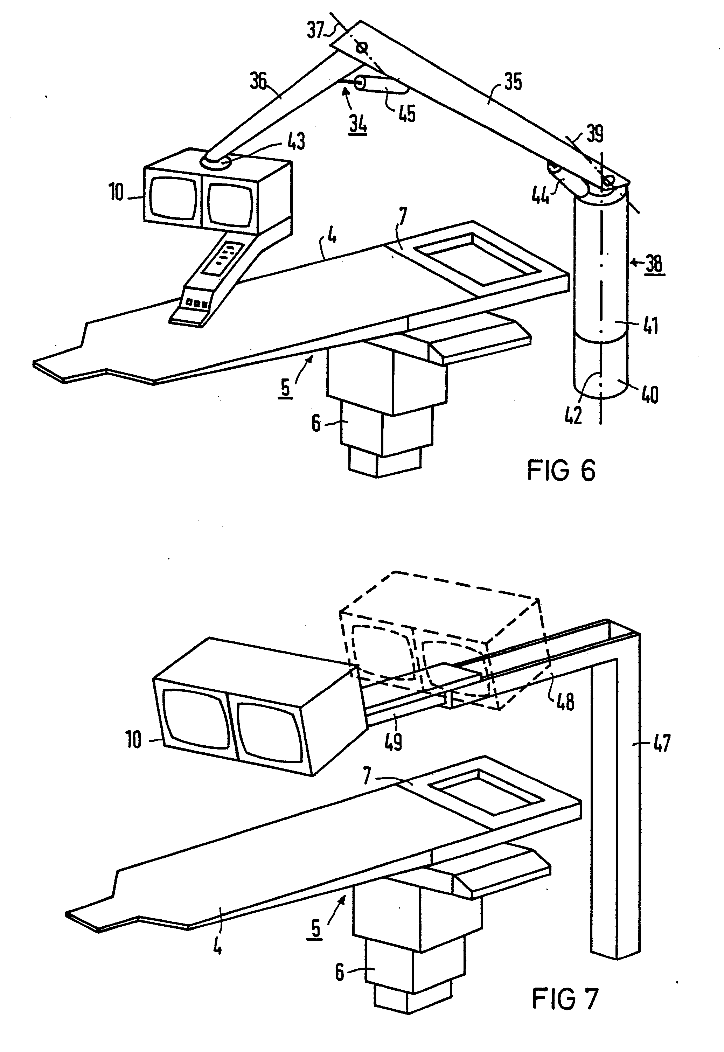

Die Fig. 6 zeigt von einem erfindungsgemäßen Röntgendiagnostikgerät wieder nur den Patientenlagerungstisch 5 und einen die Fernsehmonitore 10 tragenden insgesamt mit 34 bezeichneten Tragarm, der wie im Falle des zuvor beschriebenen Ausführungsbeispiels aus einem ersten Armabschnitt 35 und einem zweiten Armabschnitt 36 besteht, die an ihren einen Enden mittels eines nicht näher dargestellten Gelenkes diesmal um eine horizontale Gelenkachse 37 schwenkbar miteinander verbunden sind, wobei der erste Armabschnitt 35 mit seinem anderen Ende an einer am Fußboden fest montierten Säule 38 mittels einer nicht näher dargestellten Gelenkverbindung um eine horizontale Achse 39 schwenkbar angebracht ist. Die Säule 38 besteht aus den Teilen 40 und 41, von denen der Teil 40 fest mit dem Fußboden verbunden ist, während der Teil 41 relativ zu dem Teil 40 um eine vertikale Achse 42 schwenkbar ist. An dem anderen Ende des zweiten Armabschnittes 36 sind die Fernsehmonitore 10 mittels eines nicht näher dargestellten Kugelgelenkes 43 allseitig - schwenkbar ange bracht. Eine Bedienperson kann somit die Fernsehmonitore 10 mittels des Handgriffes 33, der wieder an einer mit diesen verbundenen Konsole 31 vorgesehen ist, in die gewünschte Position bringen. Außerdem können die Fernsehmonitore 10 in eine im Bereich des Fußendes 7 des Patientenlagerungstisches 5 befindliche Parkposition gebracht werden, wobei dann der erste Armabschnitt 35 etwa vertikal nach oben und der zweite Armabschnitt 36 etwa vertikal nach unten gerichtet ist. Zum Gewichtsausgleich sind übrigens Gasfedern 44 und 45 vorgesehen, von denen die eine einerseits an der Säule 38 und andererseits an dem ersten Armabschnitt 35 und die andere einerseits an dem ersten Armabschnitt 35 und andererseits an dem zweiten Armabschnitt 36 angebracht ist. Im Falle des in der Fig. 6 dargestellten Röntgendiagnostikgerätes erübrigen sich auf Grund der beschriebenen Ausbildung des Tragarmes 34 im Gegensatz zu den zuvor beschriebenen Ausführungsbeispielen besondere Maßnahmen um eine Höhenverstellbarkeit der Fernsehmonitore 10 relativ zu der Lagerungsplatte 4 zu ermöglichen.

In Fig. 7 sind von einem erfindungsgemäßen Röntgendiagnostikgerät nur der Patientenlagerungstisch 5 und der Tragarm 46, der an einer am Fußboden befestigten Säule 47 fest angebracht ist, dargestellt. Der Tragarm 46 besteht aus einem fest mit der Säule 47 verbundenen Teil 48 aus dem das die Fernsehmonitore 10 tragende Teil 49 teleskopartig ausziehbar ist. Die Fernsehmonitore 10 sind mittels eines in Fig. 7 nicht sichtbaren Kugelgelenkes allseitig schwenkbar an dem ausziehbaren Teil 49 des Tragarmes befestigt und können ausgehend von der in Fig. 7 dargestellten Position in die strichliert eingetragene Parkposition im Bereich des Fußendes 7 des Patientenlagerungstisches 5 verfahren werden. Das Röntgendiagnostikgerät nach Fig. 7 weist einen im Vergleich zu den zuvor beschriebenen Ausführungsbeispielen geringen konstruktiven Aufwand auf und ist besonders für Röntgendiagnostikgeräte geeignet, bei denen geringere Ansprüche an die Positionierbarkeit der Fernsehmonitore 10 gestellt sind.

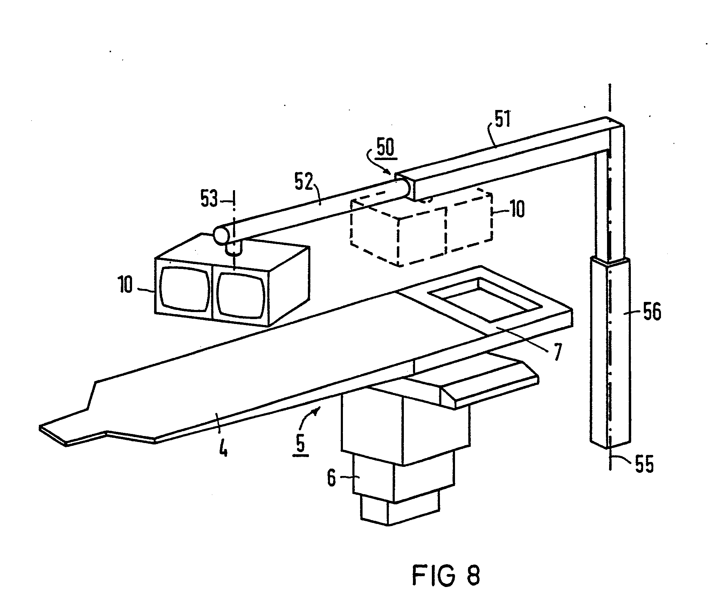

Auch im Falle des Röntgendiagnostikgerätes nach Fig. 8, von dem wieder nur der Patientenlagerungstisch 5 und ein die Fernsehmonitore 10 tragender, insgesamt mit 50 bezeichneter Tragarm dargestellt sind, ist dieser teleskopartig ausziehbar. Im einzelnen weist der Tragarm 50 einen ersten Teil 51 auf, in dem ein zweiter Teil 52, an dem die Fernsehmonitore 10 um eine vertikale Achse 53 schwenkbar angebracht sind, teleskopartig geführt ist. Der erste Teil 51 des Tragarmes 50 ist abgewinkelt und weist einen vertikal gerichteten Abschnitt 54 auf, der in einer um eine vertikale Achse 55 schwenkbar an dem Fußboden angebrachten Säule 56 zur Höhenverstellung der Fernsehmonitore 10 in Richtung der vertikalen Achse 55 längsverschieblich aufgenommen ist. Dabei kann bezüglich der vertikalen Achse 55 ein nicht dargestellter Gewichtsausgleich für den Tragarm 50 und die daran angebrachten Fernsehmonitore 10 vorgesehen sein. Auch im Falle der Ausführung nach Fig. 8 können die Fernsehmonitore 10 in eine strichliert eingetragene am Fußende 7 des Patientenlagerungstisches 5 befindliche Parkposition verfahren werden.

Sofern die Lagerungsplatte 4 des Patientenlagerungstisches 5 in Richtung ihrer Längsachse verschieblich auf dem Sockel 6 angebracht ist, sind die Säulen 38, 47 und 56 in einem solchen Abstand von dem Sockel 6 am Boden angebracht, daß die erwähnte Längsbewegung der Lagerungsplatte 4 uneingeschränkt möglich ist. Im Falle einer solchen Längsverschiebbarkeit der Lagerungsplatte 4 können außerdem Kupplungsmittel vorgesehen sein, die sicherstellen, daß die Fernsehmonitore 10 einer Längsverschiebung der Lagerungsplatte 4 synchron folgen. Ebenso können im Falle einer höhenverstellbaren Lagerungsplatte 4 weitere Kupplungsmittel vorgesehen sein, die bewirken, daß die Fernsehmonitore 10 bei einer Höhenverstellung der Lagerungsplatte 4 deren Bewegung synchron folgen. Die erwähnten Kupplungsmittel erübrigen sich im Falle des Röntgendiagnostikgerätes nach den Fig. 4 und 5, da in diesem Falle die Säule 27 an der Lagerunsplatte 4 des Patientenlagerungstisches 5 angebracht ist und die Fernsehmonitore 10 somit Bewegungen der Lagerungsplatte 4 ohnehin folgen.

**Ansprüche**

1. Röntgendiagnostikgerät mit einem an seinem einen Ende (11) einen Monitor (10) tragenden Tragarm (12, 23, 34, 46, 50) und einem Patientenlagerungstisch (5), wobei der Monitor (10) mittels des Tragarmes (12, 23, 34, 46, 50) relativ zu dem Patientenlagerungstisch (5) verstellbar ist, **dadurch gekennzeichnet,** daß der Monitor (10) mittels des Tragarmes (12, 23, 34, 46, 50), der mit seinem anderen Ende (13) an einer im Bereich des Fußendes (7) des Patientenlagerungstisches (5) befindlichen Säule (14, 27, 38, 47, 56) gehaltert ist, längs des Patientenlagerungstisches (5) verstellbar und in eine an dessen Fußende (7) befindliche Parkposition verfahrbar ist.

2. Röntgendiagnostikgerät nach Anspruch 1, **dadurch gekennzeichnet,** daß der Tragarm (23) einen ersten und einen zweiten Armabschnitt (24 bzw. 25) aufweist, die an ihren einen Enden mittels eines Gelenkes um eine vertikale Gelenkachse (26) schwenkbar miteinander verbunden sind, wobei der erste Armabschnitt (24) mit seinem anderen Ende an der Säule (27) angebracht ist, während der zweite Armabschnitt (25) an seinem anderen Ende den Monitor (10) trägt.

3. Röntgendiagnostikgerät nach Anspruch 1, **dadurch gekennzeichnet,** daß der Tragarm (34) einen ersten und einen zweiten Armabschnitt (35 bzw. 36) aufweist, die an ihren einen Enden mittels eines Gelenkes um eine horizontale Gelenkachse (37) schwenkbar miteinander verbunden sind, wobei der erste Armabschnitt (35) mit seinem anderen Ende an der Säule (38) angebracht ist, während der zweite Armabschnitt (36) an seinem anderen Ende den Monitor (10) trägt.

4. Röntgendiagnostikgerät nach einem der Ansprüche 1 bis 3, **dadurch gekennzeichnet,** daß der Tragarm (46, 50) teleskopartig ausziehbar ist.

5. Röntgendiagnostikgerät nach einem der Ansprüche 1 bis 4, **dadurch gekennzeichnet,** daß die Säule (14) relativ zu dem Patientenlagerungstisch (5) längsverschiebbar angebracht ist.

6. Röntgendiagnostikgerät nach einem der Ansprüche 1 bis 5, **dadurch gekennzeichnet,** daß der Monitor (10) schwenkbar an dem Tragarm (12, 23, 34, 46, 50) angebracht ist.

7. Röntgendiagnostikgerät nach einem der Ansprüche 1 bis 6, **dadurch gekennzeichnet,** daß der Tragarm (12, 23, 51) relativ zu dem Patientenlagerungstisch (5) höhenverstellbar ist.

8. Röntgendiagnostikgerät nach einem der Ansprüche 1 bis 7, **dadurch gekennzeichnet,** daß die Säule (27) an dem Patientenlagerungstisch (5) angebracht ist.

9. Röntgendiagnostikgerät nach einem der Ansprüche 1 bis 8, **dadurch gekennzeichnet,** daß an dem Tragarm (12) ein an seinem einen Ende ein Bedienkästchen (18) tragender Arm (19) angebracht ist, der mit seinem anderen Ende um eine im Bereich des Monitors (10) angeordnete Gelenkverbindung relativ zu dem Tragarm (12) schwenkbar ist.

10. Röntgendiagnostikgerät nach Anspruch 9, **dadurch gekennzeichnet,** daß der Arm (19) in eine Parkposition schwenkbar ist, in der er sich im wesentlichen parallel zu dem Tragarm (12) erstreckt.

**FIG 2**

14

15 20 12 13

11

10

17

19

21

18 8 4

7

5

6

**FIG 1**

1

2

14

15 12

16

10

17

8

9

19

18

4

5

3

6

**FIG 3**

4 10

16

11 15

12 20

13

14

7

19

22 18

0 274 072

86 P 3472 E

86 P 3472 E

FIG 4

FIG 5

FIG 6

FIG 7

86 P 3472 E

FIG 8

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | DE-U-8 521 246 (PICKER INTERNATIONAL) * Seite 4, Zeilen 8-12; Seite 8, Zeilen 4-8; Seite 8, Zeile 34 - Seite 9, Zeile 5; Seite 9, Zeile 29 - Seite 10, Zeile 9; Figuren 1,4 * | 1,7,9 | A 61 B 6/00 |
| A | --- | 5,6 | |
| Y | FR-A-2 321 231 (SIEMENS) * Seite 2, Zeile 25 - Seite 3, Zeile 18; Figuren 1-3 * | 1,7,9 | |
| A | --- | 2,8,10 | |
| A | DE-U-8 508 455 (SIEMENS) * Seite 1, Zeilen 7-9; Seite 2, Zeilen 4-17; Figuren * --- | 1,7-9 | |
| A | US-A-3 662 981 (H.C. HOGREBE) * Zusammenfassung; Spalte 2, Zeilen 13-19,58-72; Figuren 1-3 * --- | 3,6 | |
| A | DE-A-1 764 010 (SIEMENS) * Seite 1, Zeilen 1-5; Seite 2, Zeile 35 - Seite 3, Zeile 32; Figur 2 * --- | 5,6 | RECHERCHIERTE SACHGEBIETE (Int. Cl.4) |
| A | FR-A-1 269 786 (ALEXANDRE & CO.) * Insgesamt * --- | 1,5,6 | A 61 B F 16 M G 06 F A 47 B |
| A | US-A-4 625 731 (P.J. QUEDENS et al.) * Insgesamt * ----- | 2,6,7 | |

Der vorliegende Recherchenbericht wurde für alle Patentansprüche erstellt

| Recherchenort | Abschlußdatum der Recherche | Prüfer |
|---|---|---|
| DEN HAAG | 08-03-1988 | FERRIGNO, A. |